# FASCICULE DE BREVET EUROPEEN

(11) **EP 1 034 839 B1**
(45) Date de publication et mention de la délivrance du brevet: **24.10.2001**
(21) Numéro de dépôt: 00400503.9
(22) Date de dépôt: 24.02.2000
(51) Int. Cl.: B01J 13/04, A61K 7/00, A61K 9/51

(54) **Nanocapsules à base de polymères dendritiques**
Nanokapseln auf der Basis dendritischer Polymere
Nanocapsules based on dendritic polymers

(30) Priorité: 02.03.1999 FR 9902579
(43) Date de publication de la demande: 13.09.2000
(73) Titulaire: L'OREAL, 75008 Paris (FR)
(72) Inventeur: Simmonnet, Jean-Thierry, 75011 Paris (FR); Richart, Pascal, 75013 Paris (FR)
(74) Mandataire: Casalonga, Axel

(56) Documents cités:
- EP-A- 0 254 447
- EP-A- 0 274 961
- EP-A- 0 447 318
- WO-A-93/17060
- WO-A-96/12754
- FR-A- 2 681 248
- DATABASE WPI Section Ch, Week 8917 Derwent Publications Ltd., London, GB; Class A96, AN 89-126027 XP002116511 & JP 01 071823 A (ROHTO PHARM CO LTD), 16 mars 1989 (1989-03-16)

## Description

La présente invention concerne des nanocapsules à base de polymères dendritiques ainsi que des compositions cosmétiques et/ou dermatologiques les contenant.

L'encapsulation ou l'absorption de principes actifs liphophiles dans des particules de dimensions submicroniques est connue depuis plusieurs années et est largement utilisée en particulier dans les domaines cosmétologique et dermatologique. En effet, ces particules appelées nanoparticules sont capables de traverser les couches superficielles du *stratum corneum* et de pénétrer dans les couches supérieures de l'épiderme vivant pour y libérer le principe actif. Cette pénétration dans des couches plus profondes élargit l'espace d'action des principes actifs et les met à l'abri d'une élimination rapide par simple frottement.

Le terme de "nanoparticules" englobe principalement deux systèmes différents : des "nanosphères" constituées d'une matrice polymère poreuse dans laquelle le principe actif est absorbé et/ou adsorbé, ainsi que des "nanocapsules" ayant une structure de type noyau-enveloppe, c'est-à-dire une structure constituée d'un coeur lipidique formant ou contenant le principe actif, lequel coeur est encapsulé dans une enveloppe protectrice continue insoluble dans l'eau. La présente invention concerne uniquement ce deuxième type vésiculaire de nanoparticules, c'est-à-dire des nanocapsules à noyau lipidique entouré d'une membrane polymère.

L'encapsulation de principes actifs dans des capsules de taille submicronique permet, il est vrai, de transporter les molécules actives plus profondément dans la peau mais elle n'assure pas toujours - contrairement à ce que pourrait laisser penser cette structure "protectrice" - une stabilité suffisante du principe actif vis-à-vis des conditions physico-chimiques environnantes.

Le problème de l'instabilité du principe actif se pose en particulier pour des substances sensibles à l'oxydation, à la lumière, à des températures élevées et/ou à des pH acides ou basiques. Une telle substance très utilisée en cosmétique est par exemple le rétinol (vitamine A₁) qui est sensible à l'oxydation en particulier à pH acide.

Une approche pour stabiliser le rétinol a consisté à ajouter aux compositions le renfermant des antioxydants liphophiles et des chélatants et d'ajuster le pH de ces compositions à une valeur comprise entre 5 et 10 (WO 9 631 194).

La demanderesse a découvert que l'encapsulation dans des nanocapsules à base d'un type de polymère particulier permettait d'améliorer de manière spectaculaire la stabilité du rétinol et ceci notamment en l'absence d'agents anti-oxydants et à des pH inférieurs à 5.

Les polymères permettant d'obtenir un tel effet favorable sont des polymères appelés *polymères dendritiques.* Ces polymères ont une structure de type polyester hyperramifiée qui sera décrite plus en détail ci-après.

Ainsi, l'encapsulation du rétinol dans des nanocapsules ayant une enveloppe formée par des polymères dendritiques du type décrit ci-après confère à cette molécule active une stabilité satisfaisante, à savoir une perte d'activité inférieure à 20 % après 1 mois de conservation à 45 °C, alors que, dans des conditions équivalentes, cette même molécule encapsulée dans d'autres polymères couramment utilisés pour la nanoencapsulation (par exemple la polycaprolactone ou les dérivés de cellulose) présente une perte d'activité au moins égale à 30 % et pouvant atteindre 100%.

L'invention a donc pour objet des nanocapsules constituées
- d'un coeur lipidique formant ou contenant un principe actif lipophile et
- d'une enveloppe continue insoluble dans l'eau comprenant au moins un polymère dendritique de type polyester à fonctions hydroxyle terminales.

Elle a également pour objet des compositions cosmétiques et/ou dermatologiques contenant lesdites nanocapsules à base de polymères dendritiques.

L'invention a en outre pour objet un procédé de préparation de nanocapsules à base des polymères dendritiques ci-dessus.

D'autres objets apparaîtront à la lecture de la description et des exemples qui suivront.

Les polymères dendritiques ou dendrimères (du grec *dendron =* arbre) sont des molécules polymères "arborescentes", c'est-à-dire très ramifiées inventées par D. A. Tomalia et son équipe au début des années 90 (Donald A. Tomalia *et al*., *Angewandte Chemie, Int. Engl. Ed.,* vol. 29, n° 2, pages 138 - 175). Il s'agit de structures moléculaires construites autour d'un motif central généralement polyvalent. Autour de ce motif central sont enchaînés, en couches concentriques et selon une structure parfaitement déterminée, des motifs ramifiés d'allongement de chaîne donnant ainsi naissance à des macromolécules symétriques, monodispersées ayant une structure chimique et stéréochimique bien définie.

Les polymères dendritiques constituant l'enveloppe des nanocapsules de la présente invention sont des polymères hyperramifiés ayant la structure chimique d'un polyester et qui sont terminés par des groupements hydroxyle éventuellement modifiés par au moins un agent de terminaison de chaîne. La structure et la préparation de tels polymères est décrite dans les demandes de brevet WO-A-93/17060 et WO 96/12754.

Plus précisément, les polymères dendritiques utilisés dans les compositions de la présente invention peuvent être définis comme étant des macromolécules hautement ramifiées de type polyester, constituées
- d'un motif central dérivé d'un composé initiateur portant une ou plusieurs fonctions hydroxyle (a),
- de motifs d'allongement de chaîne dérivés d'une molécule d'allongement de chaîne portant une fonction carboxyle (b) et au moins deux fonctions hydroxyle (c),
chacune des fonctions hydroxyle (a) de la molécule centrale étant le point de départ d'une réaction de polycondensation (par estérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

On appelle un dendrimère de "génération X" un polymère hyperramifié préparé par X cycles de condensation, chaque cycle consistant à faire réagir l'ensemble des fonctions réactives du motif central ou du polymère avec un équivalent d'une molécule d'allongement de chaîne.

Le composé initiateur portant une ou plusieurs fonctions hydroxyle et formant le motif central autour duquel se construira la structure dendritique est un composé mono-, di- ou polyhydroxylé. Il est généralement choisi parmi
(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un α-alkylglycoside,
(h) un polymère polyalcoxylé obtenu par polyalcoxylation d'un des alcools (a) à (g), ayant une masse molaire au plus égale à 8000.

On peut citer à titre d'exemples de composés initiateurs préférés servant à préparer les polyesters dendritiques utilisés dans la présente invention le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

On fait réagir sur ces composés initiateurs hydroxylés formant le motif central du futur dendrimère, des molécules dites molécules d'allongement de chaîne qui sont des composés de type diol-monoacide choisis parmi
- les acides monocarboxyliques comportant au moins deux fonctions hydroxyle, et
- les acides monocarboxyliques comportant au moins deux fonctions hydroxyle dont une ou plusieurs porte(nt) un substituant hydroxyalkyle.

Des exemples préférés de tels composés sont l'acide diméthylolpropionique, l'acide α,α-bis(hydroxyméthyl)-butyrique, l'acide α,α,α-tris(hydroxyméthyl)-acétique, l'acide α,α-bis(hydroxyméthyl)-valérique, l'acide α,α-bis(hydroxy)-propionique et l'acide 3,5-dihydroxybenzoïque.

Selon un mode de réalisation particulièrement préféré de la présente invention, le composé initiateur est choisi parmi le triméthylolpropane, le pentaérythritol et un pentaérythritol éthoxylé, et la molécule d'allongement de chaîne est l'acide diméthylolpropionique.

Une partie des fonctions hydroxyle terminales des polymères dendritiques de type polyester utilisés dans les nanocapsules de la présente invention peuvent porter des substituants dérivés d'au moins un agent de terminaison de chaîne.

La fraction de ces fonctions hydroxyle terminales portant un motif de terminaison de chaîne est généralement comprise entre 1 et 90 % en moles, de préférence entre 10 et 50 % en moles rapporté au nombre total de fonctions hydroxyle terminales.

Le choix d'un agent de terminaison de chaîne approprié permet de modifier à souhait les propriétés physico-chimiques des polyesters dendritiques utilisés dans les compositions de la présente invention.

Ledit agent de terminaison de chaîne peut être choisi dans une grande variété de composés capables de former des liaisons covalentes avec les fonctions hydroxyle terminales.

Ces composés englobent notamment :
i) les acides (ou anhydrides) monocarboxyliques aliphatiques ou cycloaliphatiques saturés ou insaturés,
ii) les acides gras saturés ou insaturés,
iii) les acides monocarboxyliques aromatiques,
iv) les diisocyanates monomères ou oligomères ou des produits d'addition de ceux-ci,
v) les épihalogénhydrines,
vi) les esters glycidyliques d'un acide monocarboxylique ou d'un acide gras en C₁₋₂₄,
vii) les éthers glycidyliques d'alcools monovalents en C₁₋₂₄,
viii) les produits d'addition dérivés d'un acide mono-, di- ou polycarboxylé aliphatique ou cycloaliphatique, saturé ou insaturé, ou des anhydrides correspondants,
ix) les produits d'addition dérivés d'un acide mono-, di- ou polycarboxylé aromatique ou des anhydrides correspondants,
x) les époxydes d'un acide monocarboxylique insaturé en C₃₋₂₄ ou d'un triglycéride correspondant,
xi) les alcools monofonctionnels aliphatiques ou cycloaliphatiques, saturés ou insaturés,
xii) les alcools monofonctionnels aromatiques,
xiii) les produits d'addition dérivés d'un alcool mono-, di- ou polyfonctionnel, aliphatique ou cycloaliphatique, saturé ou insaturé, et
xiv) les produits d'addition dérivés d'un alcool mono-, di- ou polyfonctionnel aromatique.

On peut citer à titre d'exemple d'agents de terminaison de chaîne l'acide laurique, les acides gras de graines de lin, les acides gras de soja, les acides gras de suif, les acides gras d'huile de ricin déshydrogénée, l'acide crotonique, l'acide caprique, l'acide caprylique, l'acide acrylique, l'acide méthacrylique, l'acide benzoïque, l'acide para-*tert*-butylbenzoïque, l'acide abiétique, l'acide sorbinique, le 1-chloro-2,3-époxypropane, le 1,4-dichloro-2,3-époxybutane, les acides gras de soja époxydés, le maléate de l'éther diallylique de triméthylolpropane, le 5-méthyl-1,3-dioxane-5-méthanol, le 5-éthyl-1,3-dioxane-5-méthanol, l'éther diallylique de triméthylolpropane, l'éther triallylique de pentaérythritol, le triacrylate de pentaérythritol, le triacrylate de pentaérythritol triéthoxylé, le toluène-2,4-diisocyanate, le toluène-2,6-diisocyanate, l'hexaméthylène-diisocyanate ou l'isophorone-diisocyanate.

Parmi ces agents de terminaison de chaîne, on préfère en particulier l'acide caprique et l'acide caprylique ou un mélange de ceux-ci.

Les polymères dendritiques de type polyester à fonctions hydroxyle terminales et portant éventuellement des groupements de terminaison de chaîne sont connus et sont commercialisés par la société PERSTORP.

Des polymères particulièrement préférés utilisés dans la présente invention sont
- un polyester dendritique obtenu par polycondensation d'acide diméthylolpropionique sur du triméthylolpropane et exempt d'agents de terminaison de chaîne, par exemple celui commercialisé sous la dénomination "BOLTORN® H40 (TMP core)" par la société PERSTORP ;
- un polyester dendritique obtenu par polycondensation d'acide diméthylolpropionique sur du pentaérythritol polyoxyéthyléné (en moyenne 5 motifs d'oxyde d'éthylène sur chaque fonction hydroxyle), exempt d'agent de terminaison de chaîne par exemple celui commercialisé sous la dénomination "BOLTORN® H30" par la société PERSTORP ;
- un polyester dendritique de génération 3 obtenu par polycondensation d'acide diméthylolpropionique sur du pentaérythritol polyoxyéthyléné (en moyenne 5 motifs d'oxyde d'éthylène sur chaque fonction hydroxyle), dont 50 % des fonctions hydroxyle sont estérifiées par des acides en C₈₋₁₀ et notamment les acides caprique(C₁₀) et caprylique(C₈), ("BOLTORN® H30 (estérifié)" vendu par la société PERSTORP).

Parmi ces trois polymères, on préfère tout particulièrement le dernier.

Ces polymères dendritiques sont utilisés pour préparer des nanocapsules constituées d'un coeur lipidique formant ou contenant un principe actif entouré d'une enveloppe formée par ces polymères.

Le procédé de préparation de nanocapsules utilisé par la demanderesse est de préférence celui décrit dans EP-A-0 274 961. Il comprend les étapes consistant
- à dissoudre le polymère, la phase lipidique formant ou contenant le principe actif et éventuellement un agent tensioactif jouant le rôle d'agent d'enrobage dans un solvant organique approprié, c'est-à-dire miscible avec l'eau, non toxique et plus volatil que l'eau (généralement de l'acétone et/ou un alcool inférieur),
- à préparer une solution d'un agent tensioactif approprié dans l'eau (non-solvant du polymère et de la phase lipidique),
- à verser la phase organique dans la phase aqueuse tout en agitant modérément celle-ci, ce qui aboutit à la formation spontanée d'une émulsion de nanocapsules,
- puis à évaporer la phase organique et, éventuellement, une partie de la phase aqueuse, ce qui donne une suspension concentrée de nanocapsules dans une phase aqueuse.

Ce procédé de préparation implique généralement le chauffage de la phase organique et/ou de la phase aqueuse à des températures comprises entre 35 et 70 °C. Les polyesters dendritiques utilisés dans la présente invention permettent de réaliser ce procédé à température ambiante ce qui constitue un avantage important en particulier pour des substances actives thermosensibles telles que le rétinol.

L'agent tensioactif sert principalement à maîtriser la taille des nanocapsules. Il assure en effet la stabilité des nanocapsules dans l'émulsion résultant du versement de la phase acétonique dans la phase aqueuse et prévient leur coalescence.

On peut utiliser n'importe quel agent tensioactif à caractère hydrophile, qu'il soit non-ionique, anionique ou cationique. On peut citer à titre d'exemple le laurylsulfate de sodium, les composés d'ammonium quaternaire, les monoesters de sorbitanne polyoxyéthylénés ou non, les éthers d'alcools gras et de polyoxyéthylèneglycol, les condensats d'oxyde d'éthylène et d'oxyde de propylène comme le produit PLURONIC® F-68 vendu par la société BASF ou les phospholipides tels que la lécithine.

Le rapport pondéral de l'agent tensioactif aux matériaux constitutifs des nanocapsules est avantageusement compris entre 0,01 et 0,5 et de préférence voisin de 0,2.

Il est souvent souhaitable ou nécessaire de pourvoir les nanocapsules d'un enrobage dit "lamellaire". Il s'agit d'une structure organisée en un ou plusieurs feuillet(s) lipidique(s) constitué(s) chacun d'une bicouche de molécules amphiphiles semblable à celle des membranes biologiques. Cet enrobage, outre sa fonction d'ajustement de la taille des nanocapsules, améliore l'étanchéité des nanocapsules vis-à-vis d'une fuite du principe actif vers d'autres phases lipidiques de la composition.

Les agents d'enrobage sont des agents tensioactifs à caractère hydrophobe solubles dans la phase organique utilisée dans le présent procédé et qui sont capables, en présence d'eau, de former les doubles couches lipidiques décrites ci-dessus. Dans le procédé d'encapsulation de principes actifs utilisé par la demanderesse, cet agent d'enrobage est dissous dans la phase organique (acétonique/alcoolique) contenant le polymère et la phase lipidique.

On peut citer à titre d'exemple de tels agents d'enrobage les phospholipides tels que la lécithine selon la demande EP-A-447 318, certains polycondensats d'oxyde d'éthylène et d'oxyde de propylène comme les produits vendus sous la dénomination PLURONIC® par la société BASF tels que PLURONIC L121 ou sous la dénomination SYNPERONIC® par la société ICI, ou certains agents tensioactifs siliconés, tels que ceux décrits dans les documents US-A-5 364 633 et US-A-5 411 744 et utilisés dans la demande de brevet FR-A-2 742 677, par exemple ceux vendus par la société DOW CORNING sous les dénominations DC 5329, DC 7439-146, DC 2-5695 et Q4-3667.

La taille des nanocapsules à base de polymères dendritiques ainsi obtenues est avantageusement comprise entre 50 et 800 nm, de préférence entre 100 et 300 nm.

Le procédé de nanoencapsulation de la présente invention permet d'encapsuler toutes sortes de principes actifs cosmétiques ou dermatologiques liphophiles.

On peut citer à titre d'exemple les agents émollients, les anti-inflammatoires, les anti-bactériens, les anti-fongiques, les anti-viraux, les anti-séborrhéiques, les anti-acnéiques, les kératolytiques, les antihistaminiques, les anesthésiques, les agents cicatrisants, les modificateurs de la pigmentation, les filtres solaires, les piégeurs de radicaux libres, les agents hydratants, les vitamines et d'autres composés lipophiles similaires.

Selon la présente invention, le principe actif encapsulé est de préférence un principe actif lipophile sensible aux conditions physico-chimiques environnantes telles que la température, le pH, la lumière ou la présence d'agents oxydants.

On peut citer à titre d'exemples de principes actifs lipophiles préférés les vitamines telles que la vitamine A (rétinol) ou des esters de celle-ci, la vitamine E ou des esters de celle-ci tels que l'acétate de tocophérol, la vitamine D ou des dérivés de celle-ci et la vitamine F ou des dérivés de celle-ci, les carotènes tels que le β-carotène ou des dérivés de ceux-ci tels que le lykopène, et l'acide salicylique ou ses dérivés, notamment ceux décrits dans les documents FR-A-2 581 542, EP-A-378 936 et EP-A-570230, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique.

On a obtenu d'excellents résultats notamment pour l'encapsulation du rétinol (vitamine A₁), molécule très sensible à l'oxydation à pH acide, ainsi que pour les esters en C₁₋₃₀, plus particulièrement en C₁₋₆, de celui-ci, tels que l'acétate de rétinol et le propionate de rétinol.

La présente invention a également pour objet des compositions cosmétiques et/ou dermatologiques contenant, dans un support physiologiquement acceptable, les nanocapsules à base de polymères dendritiques décrites ci-dessus.

La fraction que représentent les nanocapsules dans les compositions cosmétiques et/ou dermatologiques de la présente invention est généralement comprise entre 0,1 et 30 % en poids et de préférence entre 0,5 et 15 % en poids rapporté au poids total de la composition.

Les compositions peuvent contenir, en plus des nanocapsules et de la phase aqueuse, des adjuvants cosmétiques et/ou pharmaceutiques connus tels que des corps gras, de la vaseline, des agents régulateurs de pH, des conservateurs, des agents épaississants, des colorants ou des parfums.

Bien entendu, l'homme de métier veillera à choisir ce ou ces éventuels composés supplémentaires et leur quantité, de manière à ce que les propriétés avantageuses attachées intrinsèquement à la composition cosmétique ou dermatologique conforme à l'invention ne soient pas, ou substantiellement pas, altérées par la ou les adjonctions envisagées.

Les compositions selon l'invention peuvent se présenter par exemple sous forme de sérum, de lotion, de gel aqueux, hydroalcoolique ou huileux, d'émulsion eau-dans-huile ou huile-dans-eau ou encore sous forme de dispersions aqueuses de vésicules lipidiques constituées de lipides ioniques ou non-ioniques ou d'un mélange de ceux-ci, lesquelles vésicules renferment ou non une phase huileuse.

Les exemples, donnés ci-après à titre purement illustratif et non limitatif, permettront de mieux comprendre l'invention.

### Exemple 1

### Préparation de nanocapsules à base de polyester dendritique

Dans un ballon en verre ambré d'une capacité de 500 ml, on dissout à température ambiante, sous agitation et sous atmosphère inerte :
- 1 g de polyester dendritique de triméthylolpropane et d'acide diméthylolpropionique vendu sous la dénomination BOLTORN® H40 (TMP core) par la société PERSTORP ;
- 5 g de triglycérides d'acide caprylique et d'acide caprique (phase lipidique) contenant 10 % de rétinol, et
- 1 g d'un agent tensioactif non ionique polyéthoxylé vendu sous la dénomination PLURONIC® L121 par la société BASF) dans 200 ml d'un mélange acétone/éthanol (85/15).

Dans un ballon en verre ambré d'une capacité de 11, on dissout 0,5 g d'un agent tensioactif non-ionique (polycondensat triséquencé d'oxyde d'éthylène et d'oxyde de propylène commercialisé sous la dénominationPLURONIC® F68 par la société BASF) dans 300 g d'eau à température ambiante.

On verse la phase acéto-alcoolique dans la phase aqueuse en maintenant une agitation modérée.

On évapore ensuite dans un évaporateur rotatif l'acétone et une partie de l'eau jusqu'à un volume final de 100 ml.

Cette suspension aqueuse contient des nanocapsules ayant un diamètre moyen de 230 nm.

### Exemple 2

### Préparation de nanocapsules à base de polyester dendritique

On procède de la manière décrite dans l'Exemple 1 mais en remplaçant le polyester dendritique de triméthylolpropane et d'acide diméthylolpropionique par une quantité équivalente en poids d'un polyester dendritique de pentaérythritol oxyéthyléné (en moyenne 5 motifs d'oxyde d'éthylène par fonction hydroxyle) dont 50 % des fonctions hydroxyle terminales sont estérifiées par un mélange d'acide caprylique (C₈) et d'acide caprique (C₁₀), à savoir le produit BOLTORN® H30 (estérifié) vendu par la société PERSTORP.

On obtient une suspension aqueuse de nanocapsules ayant un diamètre moyen de 202 nm.

### Exemple 3

### Essais de stabilité du rétinol encapsulé dans différents polymères

On compare la stabilité du rétinol enfermé dans les nanocapsules à base de polymères dendritiques (obtenues dans l'Exemple 2) à la stabilité de ce principe actif dans des nanocapsules à base de polymères connus que sont la polycaprolactone et l'acétobutyrate de cellulose, enrobées de différents agents d'enrobage.

Les nanocapsules contenant le rétinol sont conservées en conditionnements clos et étanches à la lumière et aux gaz sous forme de suspension aqueuse pendant un mois à 45 °C. Au bout de cette période de conservation, on évalue la perte en principe actif (rétinol) par dosage HPLC.

Les résultats obtenus sont rassemblés dans le tableau suivant.

| polymère | BOLTORN® H30 estérifié à 50 % par des acides gras en C_{8/10} | polycaprolactone | | acétobutyrate de cellulose |
|---|---|---|---|---|
| masse molaire du polymère | 4713 | 50 000 | | 30 000 |
| P_{f} du polymère (en °C) | 70 - 90 | 58 - 60 | | 155 - 165 |
| agent d'enrobage | Pluronic® L121* | lécithine | Synperonic®* | Synperonic®* |
| diamètre moyen des nanocapsules | 202 nm | 196 nm | 331 nm | 227 nm |
| pH de la composition | 4,75 | 4,2 | 4,5 | 4,9 |
| perte de principe actif après 1 mois à 45 °C | 20 % | 100 % | 41 % | 32 % |

| | | | | |
|---|---|---|---|---|
| * Synperonic® et Pluronic® sont les dénominations commerciales de polycondensats triséquencés d'oxyde de propylène et d'oxyde d'éthylène commercialisés respectivement par les sociétés ICI et BASF. | | | | |

Ces résultats montrent que l'utilisation des polymères dendritiques pour la préparation de nanocapsules améliore de manière significative la stabilité à l'oxydation du rétinol encapsulé et ceci à pH inférieur à 5.

## Revendications

1. Nanocapsules constituées
- d'un coeur lipidique formant ou contenant un principe actif lipophile et
- d'une enveloppe polymérique continue insoluble dans l'eau,
**caractérisées par le fait que** ladite enveloppe polymérique comprend au moins un polymère dendritique de type polyester à fonctions hydroxyle terminales.

2. Nanocapsules selon la revendication 1, **caractérisées par le fait que** le polymère dendritique à fonctions hydroxyle terminales est une macromolécule hautement ramifiée de type polyester, constituée
- d'un motif central dérivé d'un composé initiateur portant une ou plusieurs fonctions hydroxyle (a),
- de motifs d'allongement de chaîne dérivés d'une molécule d'allongement de chaîne portant une fonction carboxyle (b) et au moins deux fonctions hydroxyle (c),
chacune des fonctions hydroxyle (a) de la molécule centrale étant le point de départ d'une réaction de polycondensation (polyestérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

3. Nanocapsules selon la revendication 1 et 2, **caractérisées par le fait que** le composé initiateur portant une ou plusieurs fonctions hydroxyle formant le motif central est choisi parmi
(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un α-alkylglycoside, et
(h) un polymère polyalcoxylé obtenu par polyalcoxylation d'un des alcools (a) à (g), ayant une masse molaire au plus égale à 8000.

4. Nanocapsules selon la revendication 3, **caractérisées par le fait que** le composé initiateur est choisi parmi le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane et le 1,3-dioxane-5,5-diméthanol.

5. Nanocapsules selon l'une quelconque des revendications 1 à 4, **caractérisées par le fait que** la molécule d'allongement de chaîne est choisie parmi
- les acides monocarboxyliques comportant au moins deux fonctions hydroxyle, et
- les acides monocarboxyliques comportant au moins deux fonctions hydroxyle dont une ou plusieurs porte(nt) un substituant hydroxyalkyle.

6. Nanocapsule selon la revendication 5, **caractérisée par le fait que** la molécule d'allongement de chaîne est choisie parmi l'acide diméthylolpropionique, l'acide α,α-bis(hydroxyméthyl)-butyrique, l'acide α,α,α,-tris(hydroxyméthyl)-acétique, l'acide α,α-bis(hydroxyméthyl)valérique, l'acide α,α-bis(hydroxy)-propionique et l'acide 3,5-dihydroxybenzoïque.

7. Nanocapsules selon l'une quelconque des revendications 1 à 6, **caractérisées par le fait que** le composé initiateur est choisi parmi le triméthylolpropane, le pentaérythritol et un pentaérythritol polyéthoxylé, et que la molécule d'allongement de chaîne est l'acide diméthylolpropionique.

8. Nanocapsules selon l'une quelconque des revendications 1 à 7, **caractérisées par le fait qu'**une partie des fonctions hydroxyle terminales du polymère dendritique de type polyester portent des substituants dérivés d'au moins un agent de terminaison de chaîne.

9. Nanocapsules selon la revendication 8, **caractérisées par le fait que** l'agent de terminaison de chaîne est choisi parmi
i) les acides (ou anhydrides) monocarboxyliques aliphatiques ou cycloaliphatiques saturés ou insaturés,
ii) les acides gras saturés ou insaturés,
iii) les acides monocarboxyliques aromatiques,
iv) les diisocyanates monomères ou oligomères ou des produits d'addition de ceux-ci,
v) les épihalogénhydrines,
vi) les esters glycidyliques d'un acide monocarboxylique ou d'un acide gras en C₁₋₂₄,
vii) les éthers glycidyliques d'alcools monovalents en C₁₋₂₄,
viii) les produits d'addition dérivés d'un acide mono-, di- ou polycarboxylé aliphatique ou cycloaliphatique, saturé ou insaturé, ou des anhydrides correspondants,
ix) les produits d'addition dérivés d'un acide mono-, di- ou polycarboxylé aromatique ou des anhydrides correspondants,
x) les époxydes d'un acide monocarboxylique insaturé en C₃₋₂₄ ou d'un triglycéride correspondant,
xi) les alcools monofonctionnels aliphatiques ou cycloaliphatiques, saturés ou insaturés,
xii) les alcools monofonctionnels aromatiques,
xiii) les produits d'addition dérivés d'un alcool mono-, di- ou polyfonctionnel, aliphatique ou cycloaliphatique, saturé ou insaturé, et
xiv) les produits d'addition dérivés d'un alcool mono-, di- ou polyfonctionnel aromatique.

10. Nanocapsules selon la revendication 9, **caractérisées par le fait que** l'agent de terminaison de chaîne est choisi parmi l'acide laurique, les acides gras de graines de lin, les acides gras de soja, les acide gras de suif, les acides gras d'huile de ricin déshydrogénée, l'acide crotonique, l'acide caprique, l'acide caprylique, l'acide acrylique, l'acide méthacrylique, l'acide benzoïque, l'acide para-tert-butylbenzoïque. l'acide abiétique, l'acide sorbinique, le 1-chloro-2,3-époxypropane, le 1,4-dichloro-2,3-époxybutane, les acides gras de sojà époxydés, le maléate de l'éther diallylique de triméthylolpropane, le 5-méthyl-1,3-dioxane-5-méthanol, le 5-éthyl-1,3-dioxane-5-méthanol, l'éther diallylique de triméthylolpropane, l'éther triallylique de pentaérythritol, le triacrylate de pentaérythritol, le triacrylate de pentaérythritol triéthoxylé, le toluène-2,4-diisocyanate, le toluène-2,6-diisocyanate, l'hexaméthylène-diisocyanate et l'isophorone-diisocyanate.

11. Nanocapsules selon la revendication 10 dans laquelle l'agent de terminaison de chaîne est un mélange d'acide caprique et d'acide caprylique.

12. Nanocapsules selon l'une des revendications 8 à 11, **caractérisée par le fait que** la fraction de fonctions hydroxyle terminales portant un motif de terminaison de chaîne est comprise entre 1 et 90 % en moles, de préférence entre 10 et 50 % en moles rapporté au nombre total de fonctions hydroxyle terminales.

13. Nanocapsules selon l'une des revendications 1 à 11, **caractérisée par le fait qu'**elles sont entourées en outre par un enrobage lamellaire ayant une structure organisée en un ou plusieurs feuillet(s) constitué(s) chacun d'une double couche de molécules amphiphiles appelées agent d'enrobage.

14. Nanocapsules selon la revendication 13, **caractérisées par le fait que** ledit agent d'enrobage est choisi parmi les phospholipides, notamment la lécithine, les polycondensats d'oxyde de propylène et d'oxyde d'éthylène et les agents tensioactifs siliconés, capables de former des structures lamellaires.

15. Nanocapsules selon la revendication 14, **caractérisées par le fait qu'**elles ont une taille moyenne comprise entre 50 nm et 800 nm, de préférence entre 100 nm et 300 nm.

16. Nanocapsules selon l'une quelconque des revendications 1 à 15, **caractérisées par** le fait les principes actifs lipophiles encapsulés sont choisis parmi les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants et les vitamines.

17. Nanocapsules selon la revendication 16, **caractérisées par le fait que** le principe actif lipophile encapsulé est choisi parmi les principes actifs sensibles aux conditions physico-chimiques environnantes telles que la température, la lumière, le pH ou la présence d'agents oxydants.

18. Nanocapsules selon la revendication 17, **caractérisées par le fait que** le principe actif lipophile est choisi parmi les vitamines telles que la vitamine A (rétinol), la vitamine E, la vitamine D et la vitamine F et les esters et dérivés de celles-ci, les carotènes tels que le β-carotène ou les dérivés de ceux-ci tels que le lykopène, et l'acide salicylique et les dérivés de celui-ci, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique.

19. Nanocapsules selon la revendication 18, **caractérisées par le fait que** le principe actif lipophile est le rétinol ou un ester en C₁₋₃₀, plus particulièrement en C₁₋₆ de celui-ci.

20. Composition cosmétique et/ou dermatologique, **caractérisée par le fait qu'**elle contient, dans un support physiologiquement acceptable, les nanocapsules à base de polymères dendritiques selon l'une quelconque des revendications 1 à 19.

21. Composition cosmétique et/ou dermatologique selon la revendication 20, **caractérisée par le fait que** la fraction des nanocapsules est comprise entre 0,1 et 30 % en poids et de préférence entre 0,5 et 15 % en poids rapporté au poids total de la composition.

22. Composition cosmétique et/ou dermatologique selon la revendication 20 ou 21, **caractérisée par le fait qu'**elle contient en outre des adjuvants cosmétiques et/ou pharmaceutiques tels que des corps gras, de la vaseline, des agents régulateurs de pH, des conservateurs, des agents épaississants, des colorants ou des parfums.

23. Composition cosmétique et/ou dermatologique selon l'une quelconque des revendications 20 à 22, **caractérisée par le fait qu'**elle se présente sous forme d'un sérum, d'une lotion, d'un gel aqueux, hydroalcoolique ou huileux, d'une émulsion eau-dans-huile ou huile-dans-eau, d'une dispersion aqueuse de vésicules lipidiques constituées de lipides ioniques ou non-ioniques ou d'un mélange de ceux-ci, lesquelles vésicules renferment ou non une phase huileuse.

24. Procédé de préparation des nanocapsules selon l'une quelconque des revendications 1 à 19, consistant
- à dissoudre un polymère, une phase lipidique formant ou contenant un principe actif et éventuellement un agent d'enrobage dans un solvant organique miscible à l'eau approprié,
- à préparer une solution aqueuse d'un agent tensioactif approprié,
- à verser la phase organique dans la phase aqueuse tout en agitant modérément celle-ci,
- puis à évaporer la phase organique et, éventuellement, une partie de la phase aqueuse,
procédé de préparation **caractérisé par le fait que** le polymère utilisé dans la première étape est un polyester dendritique décrit dans l'une quelconque des revendications 1 à 12.

## Claims

1. Nanocapsules consisting
- of a lipid core forming or containing a lipophilic active principle, and
- of a water-insoluble continuous polymeric envelope, **characterized in that** the said polymeric envelope comprises at least one dendritic polymer of polyester type containing terminal hydroxyl functions.

2. Nanocapsules according to Claim 1, **characterized in that** the dendritic polymer containing terminal hydroxyl functions is a highly branched macromolecule of polyester type, consisting
- of a central unit derived from an initiator compound bearing one or more hydroxyl functions (a),
- of chain-extending units derived from a chain-extending molecule bearing a carboxyl function (b) and at least two hydroxyl functions (c),
each of the hydroxyl functions (a) of the central molecule being the starting point of a polycondensation reaction (polyesterification) which starts with the reaction of the hydroxyl functions (a) of the central molecule with the carboxyl functions (b) of the chain-extending molecules, and then continues by reaction of the carboxyl functions (b) with the hydroxyl functions (c) of the chain-extending molecules.

3. Nanocapsules according to Claims 1 and 2, **characterized in that** the initiator compound bearing one or more hydroxyl functions forming the central unit is chosen from
(a) a monofunctional alcohol,
(b) an aliphatic, cycloaliphatic or aromatic diol,
(c) a triol,
(d) a tetrol,
(e) a sugar alcohol,
(f) anhydro-ennea-heptitol or dipentaerythritol,
(g) an α-alkylglycoside, and
(h) a polyalkoxy polymer obtained by polyalkoxylation of one of the alcohols (a) to (g), with a molar mass of not more than 8000.

4. Nanocapsules according to Claim 3, **characterized in that** the initiator compound is chosen from ditrimethylolpropane, ditrimethylolethane, dipentaerythritol, pentaerythritol, an alkoxylated pentaerythritol, trimethylolethane, trimethylolpropane, an alkoxylated trimethylolpropane, glycerol, neopentyl glycol, dimethylolpropane or 1,3-dioxane-5,5-dimethanol.

5. Nanocapsules according to any one of Claims 1 to 4, **characterized in that** the chain-extending molecule is chosen from
- monocarboxylic acids comprising at least two hydroxyl functions, and
- monocarboxylic acids comprising at least two hydroxyl functions, one or more of which bear(s) a hydroxyalkyl substituent.

6. Nanocapsules according to Claim 5, **characterized in that** the chain-extending molecule is chosen from dimethylolpropionic acid, α,α-bis(hydroxymethyl)butyric acid, α,α,α-tris(hydroxymethyl)acetic acid, α,α-bis(hydroxymethyl)valeric acid, α,α-bis(hydroxy)propionic acid and 3,5-dihydroxybenzoic acid.

7. Nanocapsules according to any one of Claims 1 to 6, **characterized in that** the initiator compound is chosen from trimethylolpropane, pentaerythritol and a polyethoxylated pentaerythritol, and the chain-extending molecule is dimethylolpropionic acid.

8. Nanocapsules according to any one of Claims 1 to 7, **characterized in that** some of the terminal hydroxyl functions of the dendritic polymer of polyester type bear substituents derived from at least one chain-terminating agent.

9. Nanocapsules according to Claim 8, **characterized in that** the chain-terminating agent is chosen from
i) saturated or unsaturated, aliphatic or cycloaliphatic monocarboxylic acids (or anhydrides),
ii) saturated or unsaturated fatty acids,
iii) aromatic monocarboxylic acids,
iv) diisocyanate monomers or oligomers or addition products thereof,
v) epihalohydrins,
vi) glycidyl esters of a monocarboxylic acid or of a C₁₋₂₄ fatty acid,
vii) glycidyl ethers of C₁₋₂₄ monovalent alcohols,
viii) addition products derived from a saturated or unsaturated, aliphatic or cycloaliphatic mono-, di- or polycarboxylic acid, or from the corresponding anhydrides,
ix) addition products derived from an aromatic mono-, di- or polycarboxylic acid or from the corresponding anhydrides,
x) epoxides of an unsaturated C₃₋₂₄ monocarboxylic acid or of a corresponding triglyceride,
xi) saturated or unsaturated, aliphatic or cycloaliphatic monofunctional alcohols,
xii) aromatic monofunctional alcohols,
xiii) addition products derived from a saturated or unsaturated, aliphatic or cycloaliphatic mono-, di- or polyfunctional alcohol, and
xiv) addition products derived from an aromatic mono-, di- or polyfunctional alcohol.

10. Nanocapsules according to Claim 9, **characterized in that** the chain-terminating agent is chosen from lauric acid, linseed fatty acids, soybean fatty acids, tallow fatty acids, dehydrogenated castor oil fatty acids, crotonic acid, capric acid, caprylic acid, acrylic acid, methacrylic acid, benzoic acid, para-tert-butylbenzoic acid, abietic acid, sorbinic acid, 1-chloro-2,3-epoxypropane, 1,4-dichloro-2,3-epoxybutane, epoxidized soybean fatty acids, trimethylolpropane diallyl ether maleate, 5-methyl-1,3-dioxane-5-methanol, 5-ethyl-1,3-dioxane-5-methanol, trimethylolpropane diallyl ether, pentaerythrityl triallyl ether, pentaerythrityl triacrylate, triethoxylated pentaerythrityl triacrylate, toluene 2,4-diisocyanate, toluene 2,6-diisocyanate, hexamethylene diisocyanate or isophorone diisocyanate.

11. Nanocapsules according to Claim 10, in which the chain-terminating agent is a mixture of capric acid and caprylic acid.

12. Nanocapsules according to one of Claims 8 to 11, **characterized in that** the fraction of terminal hydroxyl functions bearing a chain-terminating unit is between 1 and 90 mol%, preferably between 10 and 50 mol%, relative to the total number of terminal hydroxyl functions.

13. Nanocapsules according to one of Claims 1 to 11, **characterized in that** they are also surrounded by a lamellar coat with a structure organized into one or more leaflet (s) each consisting of a bilayer of amphiphilic molecules, referred to as a coating agent.

14. Nanocapsules according to Claim 13, **characterized in that** the said coating agent is chosen from phospholipids, in particular lecithin, polycondensates of propylene oxide and of ethylene oxide, and silicone surfactants, which are capable of forming lamellar structures.

15. Nanocapsules according to Claim 14, **characterized in that** they have a mean size of between 50 nm and 800 nm, preferably between 100 nm and 300 nm.

16. Nanocapsules according to any one of Claims 1 to 15, **characterized in that** the encapsulated lipophilic active principles are chosen from emollients, anti-inflammatory agents, antibacterial agents, antifungal agents, antiviral agents, anti-seborrhoeic agents, anti-acne agents, keratolytic agents, antihistamines, anaesthetics, cicatrizing agents, pigmentation modifiers, sunscreens, free-radical scavengers, moisturizers and vitamins.

17. Nanocapsules according to Claim 16, **characterized in that** the encapsulated lipophilic active principle is chosen from active principles that are sensitive to the surrounding physicochemical conditions, such as the temperature, light, the pH or the presence of oxidizing agents.

18. Nanocapsules according to Claim 17, **characterized in that** the lipophilic active principle is chosen from vitamins such as vitamin A (retinol), vitamin E, vitamin D and vitamin F and esters and derivatives thereof, carotenes such as β-carotene or derivatives thereof such as lykopene, and salicylic acid and derivatives thereof, in particular 5-n-octanoylsalicylic acid, 5-n-decanoylsalicylic acid, 5-n-dodecanoylsalicylic acid, 5-n-octylsalicylic acid, 5-n-heptyloxysalicylic acid and 4-n-heptyloxysalicylic acid.

19. Nanocapsules according to Claim 18, **characterized in that** the lipophilic active principle is retinol or a C₁₋₃₀, more particularly C₁₋₆, ester thereof.

20. Cosmetic and/or dermatological composition, **characterized in that** it contains, in a physiologically acceptable support, the nanocapsules based on dendritic polymers according to any one of Claims 1 to 19.

21. Cosmetic and/or dermatological composition according to Claim 20, **characterized in that** the fraction of nanocapsules is between 0.1 and 30% by weight and preferably between 0.5 and 15% by weight relative to the total weight of the composition.

22. Cosmetic and/or dermatological composition according to Claim 20 or 21, **characterized in that** it also contains cosmetic and/or pharmaceutical adjuvants such as fatty substances, petroleum jelly, pH regulators, preserving agents, thickeners, dyes or fragrances.

23. Cosmetic and/or dermatological composition according to any one of Claims 20 to 22, **characterized in that** it is in the form of an aqueous, aqueous-alcoholic or oily serum, lotion or gel, a water-in-oil or oil-in-water emulsion or in the form of an aqueous dispersion of lipid vesicles consisting of ionic or nonionic lipids or of a mixture thereof, these vesicles possibly containing an oily phase.

24. Process for preparing the nanocapsules according to any one of Claims 1 to 19, which consists in
- dissolving a polymer, a lipid phase forming or containing an active principle and optionally a coating agent, in a suitable water-miscible organic solvent,
- preparing an aqueous solution of a suitable surfactant,
- pouring the organic phase into the aqueous phase while stirring the latter phase moderately,
- and then evaporating the organic phase and, possibly, some of the aqueous phase,
this preparation process being **characterized in that** the polymer used in the first step is a dendritic polyester described in any one of Claims 1 to 12.

## Patentansprüche

1. Nanokapseln, die aus
- einem Lipidkern, der den lipophilen Wirkstoff bildet oder enthält, und
- einer kontinuierlichen, in Wasser unlöslichen Polymerhülle bestehen, **dadurch gekennzeichnet, daß** die Polymerhülle mindestens ein dendritisches Polymer vom Polyestertyp mit endständigen Hydroxygruppen enthält.

2. Nanokapseln nach Anspruch 1, **dadurch gekennzeichnet, daß** das dendritische Polymer mit endständigen Hydroxygruppen ein hochverzweigtes Makromolekül vom Polyestertyp ist, das aus
- einer zentralen Einheit, die von einer Initiatorverbindung abgeleitet ist, die eine oder mehrere Hydroxygruppen (a) trägt,
- Einheiten zur Kettenverlängerung, die von einem Kettenverlängerungsmolekül abgeleitet sind, das eine Carboxygruppe (b) und mindestens zwei Hydroxygruppen (c) trägt,
besteht, wobei jede Hydroxygruppe (a) des zentralen Moleküls der Ausgangspunkt für eine Polykondensationsreaktion (Polyesterbildung) ist, die mit der Reaktion der Hydroxygruppen (a) des zentralen Moleküls mit den Carboxygruppen (b) der Kettenverlängerungsmoleküle beginnt und dann mit der Reaktion der Carboxygruppen (b) mit den Hydroxygruppen (c) der Kettenverlängerungsmoleküle fortgesetzt wird.

3. Nanokapseln nach Anspruch 1 und 2, **dadurch gekennzeichnet, daß** die Initiatorverbindung, die eine oder mehrere Hydroxygruppen trägt, die die zentrale Einheit bildet, ausgewählt ist unter:
(a) einwertigen Alkoholen,
(b) aliphatischen, cycloaliphatischen und aromatischen Diolen,
(c) dreiwertigen Alkoholen,
(d) vierwertigen Alkoholen,
(e) Zuckeralkoholen,
(f) Anhydro-ennea-heptit und Dipentaerythrit,
(g) α-Alkylglykosiden,
(h) polyalkoxylierten Polymeren, die durch Polyalkoxylierung eines der Alkohole (a) bis (g) erhalten werden, die eine Molmasse von höchsten 8000 aufweisen.

4. Nanokapseln nach Anspruch 3, **dadurch gekennzeichnet, daß** die Initiatorverbindung unter Ditrimethylolpropan, Ditrimethylolethan, Dipentaerythrit, Pentaerythrit, alkoxyliertem Pentaerythrit, Trimethylolethan, Trimethylolpropan, alkoxyliertem Trimethylolpropan, Glycerin, Neopentylglykol, Dimethylolpropan und 1,3-Dioxan-5,5-dimethanol ausgewählt ist.

5. Nanokapseln nach einem der Ansprüche 1 bis 4, **dadurch gekennzeichnet, daß** das Kettenverlängerungsmolekül ausgewählt ist unter:
- Monocarbonsäuren, die mindestens zwei Hydroxygruppen aufweisen, und
- Monocarbonsäuren, die mindestens zwei Hydroxygruppen aufweisen, von denen eine oder mehrere einen Hydroxyalkylsubstituenten tragen.

6. Nanokapsel nach Anspruch 5, **dadurch gekennzeichnet, daß** das Kettenverlängerungsmolekül unter Dimethylolpropionsäure,
α,α-Bis(hydroxymethyl)butansäure, α,α,α-Tris(hydroxymethyl)essigsäure, α,α-Bis(hydroxymethyl)valeriansäure,
α,α-Bis(hydroxy)propionsäure und der 3,5-Dihydroxybenzoesäure ausgewählt ist.

7. Nanokapseln nach einem der Ansprüche 1 bis 6, **dadurch gekennzeichnet, daß** die Initiatorverbindung unter Trimethylolpropan, Pentaerythrit und polyethoxyliertem Pentaerythrit ausgewählt ist und das Kettenverlängerungsmolekül aus der Dimethylolpropionsäure besteht.

8. Nanokapseln nach einem der Ansprüche 1 bis 7, **dadurch gekennzeichnet, daß** ein Teil der endständigen Hydroxygruppen des dendritischen Polymers vom Polyestertyp Substituenten trägt, die von mindestens einem Mittel zum Kettenabbruch abgeleitet sind.

9. Nanokapseln nach Anspruch 8, **dadurch gekennzeichnet, daß** das Mittel zum Kettenabbruch ausgewählt ist unter:
i) den gesättigten oder ungesättigten, aliphatischen oder cycloaliphatischen Monocarbonsäuren (und Anhydriden),
ii) den gesättigten oder ungesättigten Fettsäuren,
iii) den aromatischen Monocarbonsäuren,
iv) den Diisocyanat-Monomeren oder -Oligomeren und den Additionsprodukten dieser Verbindungen,
v) den Epihalogenhydrinen,
vi) den Glycidylestern einer Monocarbonsäure oder einer Fettsäure mit 1 bis 24 Kohlenstoffatomen,
vii) den Glycidylethern einwertiger C₁-C₂₄-Alkohole,
viii) den Additionsprodukten, die von einer gesättigten oder ungesättigten, aliphatischen oder cycloaliphatischen Mono-, Di- oder Polycarbonsäure oder den entsprechenden Anhydriden abgeleitet sind,
ix) den Additionsprodukten, die von einer aromatischen Mono-, Di- oder Polycarbonsäure oder den entsprechenden Anhydriden abgeleitet sind,
x) den Epoxiden einer ungesättigten C₃-C₂₄-Monocarbonsäure oder eines entsprechenden Triglycerids,
xi) den gesättigten oder ungesättigten, aliphatischen oder cycloaliphatischen einwertigen Alkoholen,
xii) den aromatischen einwertigen Alkoholen,
xiii) den Additionsprodukten, die von einem gesättigten oder ungesättigten, aliphatischen oder cycloaliphatischen einwertigen, zweiwertigen oder mehrwertigen Alkohol abgeleitet sind,
xiv) den Additionsprodukten, die von einem einwertigen, zweiwertigen oder mehrwertigen aromatischen Alkohol abgeleitet sind.

10. Nanokapseln nach Anspruch 9, **dadurch gekennzeichnet, daß** das Mittel zum Kettenabbruch unter Laurinsäure, den Fettsäuren von Leinsamen, den Sojafettsäuren, den Talgfettsäuren, den Fettsäuren von dehydriertem Ricinusöl, Crotonsäure, Caprinsäure, Caprylsäure, Acrylsäure, Methacrylsäure, Benzoesäure, p-*tert*.-Butylbenzoesäure, Abietinsäure, Sorbinsäure, 1-Chlor-2,3-epoxypropan, 1,4-Dichlor-2,3-epoxybutan, den epoxidierten Sojafettsäuren, dem Maleat des Trimethylolpropandiallylethers, 5-Methyl-1,3-dioxan-5-methanol, 5-Ethyl-1,3-dioxan-5-methanol, dem Trimethylolpropandiallylether, dem Pentaerythrittriallylether, dem Pentaerythrittriacrylat, dem Triacrylat von triethoxyliertem Pentaerythrit, Toluol-2,4-diisocyanat, Toluol-2,6-diisocyanat, Hexamethylendiisocyanat und Isophorondiisocyanat ausgewählt ist.

11. Nanokapseln nach Anspruch 10, wobei das Mittel zum Kettenabbruch ein Gemisch aus Caprinsäure und Caprylsäure ist.

12. Nanokapseln nach einem der Ansprüche 8 bis 11, **dadurch gekennzeichnet, daß** der Anteil der endständigen Hydroxygruppen, die eine Kettenabbruch-Einheit tragen, 1 bis 90 Mol-%, vorzugsweise 10 bis 50 Mol-%, bezogen auf die Gesamtzahl der endständigen Hydroxygruppen, beträgt.

13. Nanokapseln nach einem der Ansprüche 1 bis 11, **dadurch gekennzeichnet, daß** sie außerdem von einer lamellaren Hülle umgeben sind, die eine organisierte Struktur aus einer oder mehreren Schichten aufweist, die jeweils aus einer Doppelschicht amphiphiler Moleküle bestehen, die als Umhüllungsmittel bezeichnet werden.

14. Nanokapseln nach Anspruch 13, **dadurch gekennzeichnet, daß** das Umhüllungsmittel unter Phospholipiden, insbesondere Lecithin, den Polykondensaten aus Propylenoxid und Ethylenoxid, siliconhaltigen grenzflächenaktiven Stoffen, ausgewählt ist, die imstande sind, lamellare Strukturen zu bilden.

15. Nanokapseln nach Anspruch 14, **dadurch gekennzeichnet, daß** sie eine mittlere Größe von 50 bis 800 nm, vorzugsweise 100 bis 300 nm aufweisen.

16. Nanokapseln nach einem der Ansprüche 1 bis 15, **dadurch gekennzeichnet, daß** die eingekapselten lipophilen Wirkstoffe unter Emollientien, entzündungshemmenden Mitteln, antibakteriellen Mitteln, Antimykotika, antiviralen Mitteln, Antiseborrhöika, Antiaknemitteln, Keratolytika, Antihistaminika, Anästhetika, wundheilenden Mitteln, Mitteln zur Modifizierung der Pigmentierung, Sonnenfiltern, Radikalfängern, Hydratisierungsmitteln und Vitaminen ausgewählt sind.

17. Nanokapseln nach Anspruch 16, **dadurch gekennzeichnet, daß** der veingekapselte lipophile Wirkstoff unter den Wirkstoffen ausgewählt ist, die gegenüber den physikalisch-chemischen Einflüssen der Umwelt, wie Temperatur, Licht, pH-Wert oder dem Vorhandensein von Oxidationsmitteln, empfindlich sind.

18. Nanokapseln nach Anspruch 17, **dadurch gekennzeichnet, daß** der lipophile Wirkstoff unter Vitaminen, wie Vitamin A (Retinol), Vitamin E, Vitamin D und Vitamin F, und deren Estern und Derivaten, den Carotinen, wie β-Carotin, oder den Carotinderivaten, wie Lycopin, und Salicylsäure und den Salicylsäurederivaten, insbesondere 5-(n-Octanoyl)-salicylsäure, 5-(n-Decanoyl)-salicylsäure, 5-(n-Dodecanoyl)-salicylsäure, 5-(n-Octyl)-salicylsäure, 5-(n-Heptyloxy)-salicylsäure und 4-(n-Heptyloxy)-salicylsäure, ausgewählt ist.

19. Nanokapseln nach Anspruch 18, **dadurch gekennzeichnet, daß** es sich bei dem lipophilen Wirkstoff um Retinol oder eine C₁-C₃₀-Ester, insbesondere C₁-C₆-Ester, des Retinols handelt.

20. Kosmetische und/oder dermatologische Zusammensetzung, **dadurch gekennzeichnet, daß** sie in einem physiologisch akzeptablen Träger die Nanokapseln auf der Basis dendritischer Polymere nach einem der Ansprüche 1 bis 19 enthält.

21. Kosmetische und/oder dermatologische Zusammensetzung nach Anspruch 20, **dadurch gekennzeichnet, daß** der Anteil der Nanokapseln im Bereich von 0,1 bis 30 Gew.-% und vorzugsweise 0,5 bis 15 Gew.-%, bezogen auf das Gesamtgewicht der Zusammensetzung, liegt.

22. Kosmetische und/oder dermatologische Zusammensetzung nach Anspruch 20 oder 21, **dadurch gekennzeichnet, daß** sie außerdem kosmetische und/oder pharmazeutische Hilfsstoffe enthält, wie Fettsubstanzen, Vaseline, Mittel zur Einstellung des pH-Wertes, Konservierungsmittel, Verdickungsmittel, Färbemittel oder Parfüms.

23. Kosmetische und/oder dermatologische Zusammensetzung nach einem der Ansprüche 20 bis 22, **dadurch gekennzeichnet, daß** sie in Form eines Serums, einer Lotion, eines wäßrigen, wäßrigalkoholischen oder öligen Gels, einer Wasser-in-Öl-Emulsion oder Öl-in-Wasser-Emulsion, einer wäßrigen Dispersion von Lipidvesikeln vorliegt, die aus ionischen oder nichtionischen Lipiden oder einem Gemisch dieser Lipide bestehen, wobei die Lipidvesikeln gegebenenfalls eine Ölphase enthalten.

24. Verfahren zur Herstellung der Nanokapseln nach einem der Ansprüche 1 bis 19, das folgende Schritte umfaßt:
- Auflösen eines Polymers, einer Lipidphase, die einen Wirkstoff darstellt oder enthält, und gegebenenfalls eines Umhüllungsmittels in einem geeigneten, mit Wasser mischbaren organischen Lösemittel,
- Herstellen einer wäßrigen Lösung eines geeigneten grenzflächenaktiven Stoffs,
- Eingießen der organischen Phase in die wäßrige Phase unter leichtem Rühren der wäßrigen Phase,
- Verdampfen der organischen Phase und gegebenenfalls eines Teils der wäßrigen Phase,
wobei das Verfahren **dadurch gekennzeichnet ist, daß** das in dem ersten Schritt verwendete Polymer ein in einem der Ansprüche 1 bis 12 beschriebener dendritischer Polyester ist.
